# EUROPÄISCHE PATENTSCHRIFT

(11) **EP 0 491 310 B1**
(45) Veröffentlichungstag und Bekanntmachung des Hinweises auf die Patenterteilung: **09.10.1996**
(21) Anmeldenummer: 91121496.3
(22) Anmeldetag: 14.12.1991
(51) Int. Cl.: G01N 33/564, G01N 33/543, G01N 33/58, G01N 33/68

(54) **Verfahren zur Bestimmung von Antikörpern gegen Lipocortine**
Method for the determination of antibodies against lipocortine
Procédé pour la détermination d'anticorps contre la lipocortine

(30) Priorität: 17.12.1990 DE 4040306
(43) Veröffentlichungstag der Anmeldung: 24.06.1992
(73) Patentinhaber: BEHRINGWERKE Aktiengesellschaft, 35001 Marburg (DE)
(72) Erfinder: Kraus, Michael, Dr., W-3550 Marburg (DE); Römisch, Jürgen, Dr., W-3550 Marburg (DE)

(56) Entgegenhaltungen:
- EP-A- 0 292 810
- ANNALS OF THE RHEUMATIC DISEASES Bd. 48, Nr. 10, Oktober 1989, LONDON Seiten 843 - 850; GOULDING ET AL.: 'Autoantibodies to recombinant lipocortin-1 in .... ..'
- BIOL. CHEM. HOPPE-SEYLER Bd. 371, Mai 1990, BERLIN Seiten 383 - 388; RÖMISCH ET AL.: 'Purification and characterization of six annexins ......'
- BULLETIN OF THE WORLD HEALTH ORGANIZATION Bd. 53, Nr. 1, 1976, GENEVE Seiten 55-65. Voller et al.

## Beschreibung

Die Erfindung betrifft ein verfahren zur Bestimmung von Antikörpern gegen Lipocortine (Annexine) in einer Körperflüssigkeit einer Spezies, unter Verwendung von an eine Festphase gebundenen Proteinen aus der Lipocortinfamilie und eines markierten bioaffinen Bindungspartners, der gegen einzelne oder mehrere Klassen von Immunglobulinen dieser Spezies gerichtet ist.

Entzündliche, autoimmunologisch begründete Erkrankungen sind oft begleitet von einer Disregulation des Immunsystems, die zur Bildung von Autoantikörpern führen kann, die nicht ursächlich mit der Pathogenese der Erkrankung in Zusammenhang stehen. Diagnostisch können solche sekundären Antikörper zur Differentialdiagnose herangezogen werden. Sekundäre Autoantikörper können zum anderen jedoch auch zu Nebenwirkungen führen, die für die Therapie von Bedeutung sind. So verursacht die Anlagerung von Immunkomplexen an Zelloberflächen mit Komplementbindung und nachfolgender Komplementaktivierung in der Gefäßwand Vaskulitiden, im Herzmuskel Karditis und in den Nierentubuli Glomerulonephritis.

Bei rheumatischen Erkrankungen (Systemischer Lupus erythematodes, rheumatischer Arthritis und Dermatomyositis) wurden solche sekundären Autoantikörper gegen Proteine aus der Lipocortinfamilie beschrieben (Hirata, F. et al., Proc. Natl. Acad. Sci. USA, 78, 3190-3194, 1981).

Diese Lipocortinfamilie umfaßt zur Zeit sechs sehr gut charakterisierte Proteine, die als PP4, PP4-X, PAP III, p68, Lipocortin I und II (oder entsprechend der neuen Nomenklatur als Lipocortin bzw. Annexin V,IV, III, VI, I und II) bezeichnet werden. Lipocortine regulieren u. a. die Freisetzung von Arachidonsäure und somit die Bereitstellung von Entzündungsmediatoren - die Lipocortine wirken antiinflammatorisch.

Da diese Proteine keine "leader-Sequenz" besitzen, wurden sie vor allem intrazellulär in höheren Konzentrationen nachgewiesen, jedoch nur in Spuren in Körperflüssigkeiten. Lipocortine wurden aber auch auf Zelloberflächen gefunden und es konnte gezeigt werden, daß sie auch extrazellulär eine Entzündungshemmung bewirken können.

Bei chronischen Entzündungen besteht aber die Gefahr einer Bildung von Autoantikörpern gegen Lipocortine, die dann zur Cortikoidresistenz führt. Die Bestimmung von Autoantikörpern gegen Lipocortine sollte daher bei der Wahl der Therapieform, insbesondere bei längerfristiger Anwendung von Cortikoiden, ein wichtiges Entscheidungskriterium darstellen.

Bekannt ist ein Verfahren, Autoantikörper gegen Lipocortin I in Serum mittels ELISA zu bestimmen (Goulding et al., Ann. Rheum. Dis. 48, 843-850, 1989). Dieses Verfahren besitzt jedoch große Nachteile. So werden zum einen nur Antikörper gegen ein Protein (Lipocortin I) aus der Lipocortinfamilie erfaßt. Zum anderen ist die Empfindlichkeit gering und der Hintergrund durch unspezifische Bindung in Sera von Normalprobanden sehr hoch, so daß insbesondere Autoantikörper der IgG-Klasse kaum erfaßt werden. Schließlich konnte nicht gezeigt werden, daß Rheumafaktor-haltige Seren, die gerade bei der indizierten Patientengruppe Zu erwarten sind, richtig bestimmt werden. Dieses Verfahren ist daher als Screening Test für die klinische Routine wenig geeignet.

Es stellte sich daher die Aufgabe, eine schnelle und einfach durchzuführende immunologische Methode zur Identifizierung von Antikörpern gegen diese Lipocortin-Proteine zu entwickeln.

Es wurde nun gefunden, daß in heterogenen Immunoassays durch geeignete Beschichtungsproteine und -verfahren, Wahl des Proben- und Konjugatpuffers, sowohl die unspezifische Bindung von Antikörpern an mit Lipocortinen beschichteten Festphasen, als auch der Einfluß von Rheumafaktor stark reduziert werden kann. Eine zuverlässige diagnostische Aussage ist nur zu erwarten, wenn das Vorhandensein von Antikörpern gegen alle Lipocortine geprüft wird.

Gegenstand der Erfindung ist daher ein heterogener Immunoassay zur Bestimmung von Antikörpern gegen Lipocortine, wobei als an die Festphase gebundener, bioaffiner Bindungspartner ein Gemisch aus den bekannten Lipocortinen verwendet wird.

Heterogene Enzymimmunoassays sind an sich dem Fachmann bekannt. Sie können zum Nachweis von Antigenen und Antikörpern dienen und additiv, wie z. B. ein Sandwich-Immunoassay, oder kompetitiv sein.

Die zum Nachweis des gebundenen Analyten dienenden bioaffinen Bindungspartner sind z. B. mit einem Radioisotop, einer fluoreszierenden oder chemilumineszenten Substanz oder vorzugsweise mit einem Enzym markiert, um in bekannter Weise den Bindungsnachweis zu führen.

Markerenzyme für Enzymimmunoassays als solche sind literaturbekannt, bevorzugterweise werden alkalische Phosphatase, β-Galaktosidase und Meerrettich-Peroxidase, besonders bevorzugterweise wird Meerrettich-Peroxidase verwendet.

Festphasen für heterogene Enzymimmunoassays sind dem Fachmann an sich bekannt, bevorzugterweise werden Formkörper, wie z. B. flächenförmige Testelemente in denen die Festphase matrixförmig, wie z. B. ein Vlies, Membranfilter oder netzförmig ist, Röhrchen, Näpfchen, Kugeln, Sterne o.ä. und Mikropartikel (Korngröße < 1000 nm) wie z. B. Latexpartikel und magnetisch anziehbare Partikel verwendet.
Besonders bevorzugt sind dabei Näpfchen in Form von Mikrotitrationsplatten, Latexpartikel und magnetisch anziehbare Partikel.
Ganz besonders bevorzugt sind Mikrotitrationsplatten.

Materialen für Festphasen sind dem Fachmann bekannt.

Als Bindungspartner an der Festphase werden bevorzugt Proteine aus der Lipocortinfamilie verwendet, die humanen Ursprungs sind (z. B. nach Römisch et al. Biol. Chem. Hoppe-Seyler 371, 383 - 388, 1990). Zudem können als Bindungspartner auch gentechnologisch hergestellte Lipocortine Verwendung finden, die von pro- oder eukaryontischen Zellen exprimiert werden (siehe z. B. Römisch et al., Biochem.J. 272, s. 223 - 229 (1990)).

Bei der Durchführung von heterogenen Immunoassays werden verschiedene, dem Fachmann an sich bekannte Lösungen, u. a. zum Waschen und Verdünnen, gebraucht, die u. a. Puffer, Detergenzien und Neutralproteine enthalten.

Diese Substanzen finden auch Verwendung in den Lösungen, die für die Beschichtung, einschließlich aller dazugehörigen Waschschritte verwendet werden.

Puffersysteme zur Verwendung in Enzymimmunoassays sind dem Fachmann bekannt. Dem Fachmann ist auch bekannt, daß die Art des jeweils verwendeten Puffersystems vom zu erzielenden pH abhängt.

Detergenzien zur Verwendung in Lösungen für heterogene Enzymimmunoassays sind dem Fachmann ebenfalls bekannt (siehe z. B. Voller, A. et al. Bull. World Health Organ. 53, 55 - 65 (1976)), bevorzugterweise verwendet werden dabei nichtionische und zwitterionische Detergenzien, besonders bevorzugt sind Polyoxyethylene, ganz besonders bevorzugt ist ^{R}Tween 20.

Neutralproteine zur Verwendung in Enzymimmunoassays sind dem Fachmann bekannt, bevorzugt verwendet werden z. B. Serumalbumine, Gelatine, chemisch modifizierte Gelatine, wie z. B. Polygelin, und Milchproteine, wie z. B. Lactoferrin, besonders bevorzugt sind Human- oder Rinderserumalbumin, Polygelin und Lactoferrin, ganz besonders bevorzugt sind Polygelin und Lactoferrin.

Gegenstand der Erfindung ist ferner ein Verfahren zur Beschichtung von Festphasen für heterogene Immunoassays zur Bestimmung von Antikörpern gegen Lipocortine, wobei die Beschichtung mit einzelnen oder Gemischen von Proteinen aus der Lipocortinfamilie in einen pH Bereich von 5 - 10, bevorzugterweise im pH Bereich von 5 - 7, besonders bevorzugt bei pH 5,5 erfolgt.

Zu einer Optimierung des Verhältnisses vom Signal der pathologischen Probe zur Normalprobe wird man bevorzugterweise eine bestimmte Kombination von pH-Wert und Zusätzen an divalenten Kationen wählen.

Bevorzugt wird dabei ein Verfahren, worin die Beschichtung der Festphase mittels einer in einem Bereich von pH 5 - 10 gepufferten Lösung von Proteinen aus der Lipocortinfamilie in Gegenwart von divalenten Kationen erfolgt, speziell von Ca²⁺,bevorzugterweise 0,1-100 mmol/l, besonders bevorzugterweise 1-10 mmol/l.

Gegebenenfalls können die Proteine aus der Lipocortinfamilie einzeln oder in Gruppen auf separate Festphasen aufgebracht werden, die dann, getrennt oder zusammen, mit der Probe inkubiert werden.

In einem bevorzugten Verfahren erfolgt die Beschichtung der Festphase, wie schon erwähnt, durch Bindung an einen an die Festphase adsorptiv oder kovalent gebundenen Antikörper, wobei dieser Antikörper aus einer anderen Spezies stammen muß als der, aus deren Körperflüssigkeit Antikörper gegen die Lipocortine bestimmt werden soll, um Kreuzreaktionen mit dem Sekundär-Antikörper zu vermeiden, der zur Markierung der aus der Körperflüssigkeit adsorbierten Antikörper verwendet wird.

Die Eigenschaften dieses Sekundär-Antikörpers sind nun so auszuwählen, daß er mit dem Antikörper der Probe reagiert, nicht aber mit dem für die Beschichtung gewählten Antikörper oder den Lipocortinen. Wenn man Antikörper gegen Lipocortine in menschlichem Serum bestimmen will, so ist ein Antikörper gegen Humanantikörper geeignet. Es ist für die Erfindung unwesentlich, ob es sich um einen polyklonalen oder monoklonalen Antikörper handelt. Wichtig ist, daß er nicht mit den Lipocortinen, die für die Beschichtung verwendet wurden, kreuzreagiert.

Am einfachsten wird diese Bedingung dadurch erfüllt, daß Antikörper und Lipocortine von derselben Spezies verwendet werden. Desweiteren kann eine unspezifische Reaktion der zur Markierung verwendeten Antikörper mit den zur Beschichtung verwendeten Antikörpern durch die Wahl eines geeigneten Puffermediums verringert werden, das in bevorzugter Weise außer den Puffersubstanzen und Zusätzen wie Detergenzien und Proteine, Antikörper enthält, die besonders bevorzugt von derselben Spezies stammen, aus der die zur Markierung verwendeten Antikörper gewonnen wurden und die nicht mit dem zur Beschichtung verwendeten Antigen reagieren.

In Zusammenhang mit dieser Optimierung wird die Festphase in einem bevorzugten Verfahren mit dem Fachmann bekannten Agenzien nachbeschichtet, wobei bevorzugt Rinderserumalbumin eingesetzt wird.

Der markierte Bindungspartner kann ein gegen einzelne Gruppen von Immunglobulinklassen gerichteter Antikörper sein.

Bevorzugt wird ein Verfahren, worin der markierte Bindungspartner ein gegen IgG, IgM oder die schwere Kette von IgG und IgM gerichteter Antikörper ist.

Diese Antikörper werden vorzugsweise durch Immunisieren einer Spezies, die vorzugsweise nicht die Spezies ist, von der die Körperflüssigkeit stammt, mit Immunglobulinen der zu untersuchenden Spezies, gewonnen und nach dem Fachmann bekannten Verfahren markiert.

Gegenstand der Erfindung ist auch ein Verfahren, worin die zu untersuchende Körperflüssigkeit in einem Puffermedium verdünnt wird, das bevorzugterweise Reagenzien enthält, die die Bindung von in der Körperflüssigkeit eventuell enthaltenen Rheumafaktoren an Antikörper dieser Körperflüssigkeit unterdrücken.

Werden menschliche Körperflüssigkeiten zur Untersuchung verwendet, so können in einer speziellen Form bei der Bestimmung von Antikörpern gegen Lipocortine aus der IgM-Fraktion oder der GesamtImmunglobulinfraktion Proben, die hohe Konzentrationen an Rheumafaktoren (humane anti-human-IgG Antikörper) und eine hohe Konzentration an Antikörpern aus der IgG-Fraktion besitzen, die gegen das oder die auf der Oberfläche beschichteten Lipocortine gerichtet sind, in einem Puffermedium verdünnt werden, das geeignete Antikörper enthält, an die diese Rheumafaktoren adsorbieren und nicht mehr mit den an den Lipocortinen adsorbierten Antikörpern der IgG-Fraktion reagieren können.

Bevorzugterweise wird dazu als Zusatz zum Puffermedium eine Globulinfraktion von Kaninchen anti-Schaferythrozyten Antikörpern verwendet.

Eine bevorzugte Ausführungsform des erfindungsgemäßen Bestimmungsverfahrens ist folgende:

Die Näpfchen einer Mikrotitrationsplatte werden mit einer gepufferten Lösung eines Gemisches aus den Lipocortinen PP4, PP4-X, PAP III, Lipocortin I und Lipocortin II bei einem pH von 5 bis 7 in Gegenwart von 0,1 - 100 mmol/l, bevorzugterweise 1 - 10 mmol/l, besonders bevorzugterweise 5 mmol/l CaCl₂ beschichtet. Die Nachbeschichtung erfolgt mit Rinderserumalbumin.
Die beschichteten Mikrotitrationsplatten können getrocknet und unter geeigneten Bedingungen z. B. in kunststoffbeschichteter Aluminiumfolie eingeschweißt über einen längeren Zeitraum ohne Aktivitätsverlust gelagert werden. Zur Beschichtung wird eine Probe bzw. ein Kontrollserum in die Näpfchen pipettiert und nach einer definierten Inkubationszeit wieder abgesaugt.

Zur Bestimmung der an der Festphase immobilisierten Antikörper wird ein Peroxidase-Antikörper (IgG und/oder IgM) Konjugat pipettiert und nach einer weiteren, definierten Inkubationszeit abgesaugt.
Zur photometrischen Bestimmung des gebundenen Konjugats wird eine Substratlösung (z. B. OPD oder TMB) einpipettiert und die Reaktion nach einer definierten Inkubationszeit mit Schwefelsäure abgestoppt. Die Extinktion wird im Photometer bestimmt. Nach dem Absaugen kann jeweils noch ein oder mehrere Waschritte durchgeführt werden.

In einer weiteren bevorzugten Ausführungsform wird als Festphase ein superparamagnetischer Partikel und als Detektionssystem ein chemolumineszentes Label, wie z. B. in EP 0 330 050 beschrieben, verwendet.

Besonders bevorzugt sind die in den Beispielen angegebenen Ausführungsformen.

Bestandteil der Offenbarung sind auch die Ansprüche.

Die folgenden Beispiele dienen lediglich der Erläuterung der Erfindung, sie schränken sie in keiner Weise ein.

### Beispiel 1

**Beschichtung von Mikrotitrationsplatten mit Lipocortinen und die Auswirkung verschiedener Bedingungen auf die Reaktion mit Serum und Plasma aus einem Pool von Normalspendern.**

Das Lipocortin PP4 wurde in einer Konzentration von 5mg/l (Herstellung der Lipocortine nach Römisch et al. (1990) Biol. Chem. Hoppe-Seyler 371, 383-388) in den folgenden Puffersystemen gelöst: 0,01 mol/l Acetatpuffer, pH 5,5; 0,01 mol/l HEPES (N-[2-Hydroxyethyl)piperazin-N'-[2-Ethansulfonsäure]), pH 7,5. Die Lösungen wurden entweder mit EDTA (Ethylendiamintetraessigsäure) oder mit CaCl₂ in einer Konzentration von 0,005 mol/l versetzt. 0,125 ml wurden in jede Vertiefung von Mikrotitrationsplatten (Fa. Nunc) gefüllt. Nach Inkubation über Nacht wurde mit 0,05 mol/l Tris/HCL-Puffer, pH 7,4 mehrmals gewaschen und die Mikrotitrationsplatten im Trockenschrank über Kieselgel bei Raumtemperatur getrocknet. Nach dem Trocknen wurden sie in Aluminium-beschichteten Kunststoffbeuteln bis zur späteren Verwendung luft- und feuchtigkeitsdicht verschlossen.

Humanes Plasma sowie Serum aus einem Pool von Normalspendern wurde in einem Tris-Puffer (0,05 mol/l Tris, pH 7,4, enthaltend Rinderserumalbumin 1% (w/w), ®Tween 20 0,5% (w/w) und NaCl 0,045 mol/l) im Verhältnis 1:5, 1:10 und 1:20 verdünnt. In die mit Lipocortin beschichteten Vertiefungen der Mikrotitrationsplatten wurden nacheinander 0,1 ml der vorverdünnten Proben pipettiert und 1 Stunde bei Raumtemperatur inkubiert. Anschließend wurde die Lösung abgesaugt und dreimal mit Puffer (z.B. Enzygnost-Waschpuffer der Behringwerke, Bestell-Nr. OSNK) gewaschen. Es wurde nun in jede Vertiefung 0,1 ml eines in obigem Tris-Puffer 1:51 verdünnten Antikörper-Konjugats (z.B. anti-Human IgG-Peroxidase Konjugat, Chg.B. 63 AP 002 A; oder anti-Human IgM-Peroxidase Konjugat, Chg. B. 88041 A, Behringwerke Marburg) pipettiert und 1 Stunde bei Raumtemperatur inkubiert. Es wurde wiederum dreimal gewaschen und je 0,1 ml Substratlösung (z.B. Substratlösung o-Phenylendiamin, Behringwerke, Bestell-Nr. OSNK) eingefüllt. Nach 30 min bei Raumtemperatur wurde die Reaktion mit je 1 ml 0,5 normaler Schwefelsäure abgestoppt und die Extinktion bei 490 nm gegen eine Referenzwellenlänge von 630 nm in einem ELISA-Photometer (z.B. Fa. Titertek) gemessen.

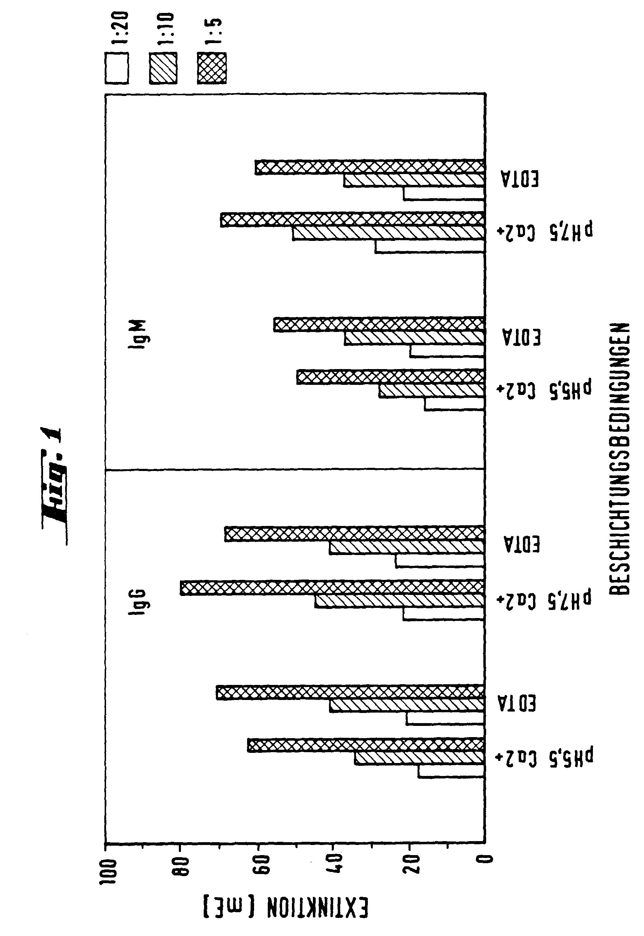

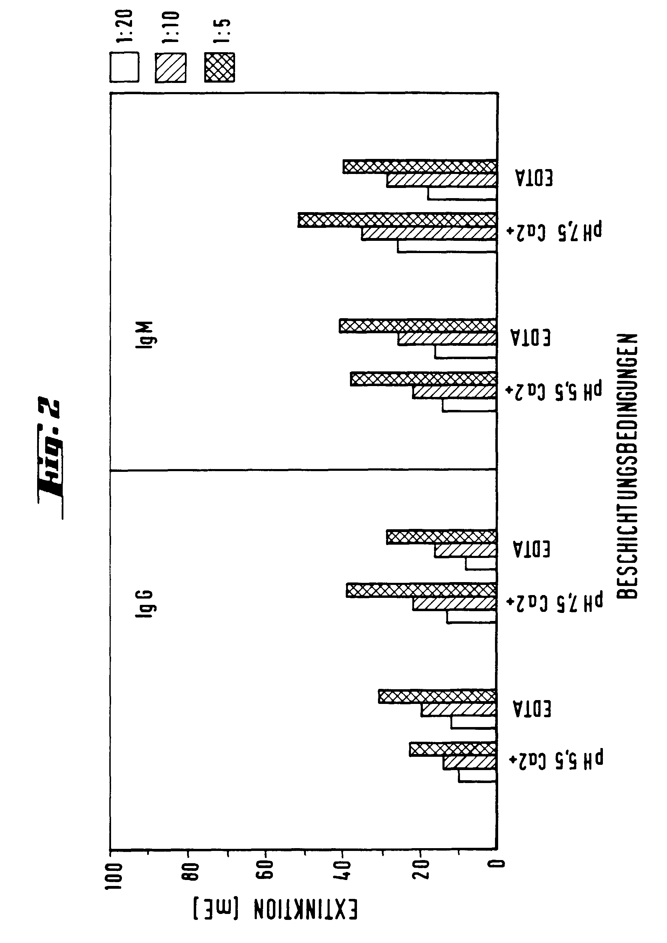

Die unter den unterschiedlichen Beschichtungsbedingungen bestimmten Extinktionen bei der IgG- bzw. der IgM-Bestimmung in Serum bzw. Plasma sind in der Figur 1 bzw. 2 dargestellt. Es ist ersichtlich, daß unter allen oben aufgeführten Beschichtungsbedingungen humane Antikörper, sowohl vom IgG- als auch vom IgM-Typ, in Serum und Plasma von Normalspendern auf Lipocortin beschichteten Platten nachweisbar sind. Die Abnahme des Signals mit zunehmendem Verdünnungsgrad der eingesetzten Probe zeigt, daß dies nicht auf unspezifische Bindung des Konjugats, sondern auf die Adsorption von IgG und IgM aus der Probe zurückzuführen ist. Dieses, in der Folge als Hintergrund bezeichnete Signal war unter den gewählten Versuchsbedingungen in Plasma stärker ausgeprägt als in Serum und bei Antikörpern vom IgM-Typ höher als bei solchen vom IgG-Typ. Der Hintergrund war in diesem Beispiel hinsichtlich der IgG- als auch der IgM-Bestimmung im sauren Beschichtungssystem durchweg niedriger als im neutralen. Im sauren Milieu konnte der Hintergrund durch die Beschichtung in Gegenwart von zweiwertigen Kationen (z. B. Ca²⁺) weiter reduziert werden.

### Beispiel 2

### Nachbeschichtung der mit Lipocortinen beschichteten Mikrotitrationsplatten und Auswirkung auf das Hintergrundsignal

Mikrotitrationsplatten wurden wie in Beispiel 1 beschrieben bei pH 5,5, 7,5 und 9,5 unter Zusatz von 0,005mol/l CaCl₂ mit einem Gemisch von Lipocortin I und II (5 bzw. 1,25 mg/l) beschichtet. Nach mehrmaligem Waschen in 0,05 mol/l Tris/HCL-Puffer pH7,4, wurde in jede Vertiefung 0,125ml 1%(w/w) Lösungen folgender Proteine des bei der Lipocortinbeschichtung verwendeten Puffers pipettiert: Rinderserumalbumin, Ovalbumin, Gelatin, Fötales Kälberserum, sowie 0,1% Kaninchen IgG und Beschichtungspuffer ohne Zusatz von Protein. Nach 3-stündiger Inkubation bei Raumtemperatur wurden die Mikrotitrationsplatten mehrmals wie unter Beispiel 1 beschrieben gewaschen und getrocknet. Die Bestimmung des Hintergrundsignals erfolgte wie unter Beispiel 1 beschrieben mit je 0,1 ml einer 1:10 Verdünnung eines humanen Serumpools von Normalspendern.

Die Ergebnisse in **Tabelle 1** zeigen, daß das Hintergrundsignal ganz entscheidend, sowohl bei der Bestimmung von Antikörpern vom IgG-Typ als auch vom IgM-Typ, durch die Art des für die Nachbeschichtung gewählten Proteins und Beschichtungspuffers reduziert werden kann. In diesem Beispiel erwies sich RSA besonders geeignet bei der Bestimmung von Antikörpern vom IgG-Typ, RSA und fötales Kälberserum bei der IgM-Bestimmung.

### Beispiel 3

### Abhängigkeit des Meßsignals von der Konzentration des Antigens bei der Plattenbeschichtung

Mikrotitrationsplatten wurden mit PP4 und PP4-X in einem Acetatpuffer (0,01 mol/l Acetat, pH 5,5; 0,005mol/l CaCl₂) in den folgenden Konzentrationen beschichtet: 39, 78, 156, 313, 625, 1250, 2500 und 5000µg/l. Die Nachbeschichtung erfolgte wie in Beispiel 2 beschrieben mit einer 1% Rinderserumalbuminlösung in dem gleichen Acetat-puffer, der zur Antigenbeschichtung verwendet wurde. Nach Waschen und Trocknen der Platten wurde humanes Serum aus einem Normalspenderpool und pathologische Sera in einem Tris-Puffer (0,05 mol/l Tris, pH 7,4, enthaltend Rinderserumalbumin 3% (w/w) ®Tween 20 0,5% (w/w) und NaCl 0,045 mol/l) im Verhältnis 1:20 verdünnt. Als pathologisches Serum zur Bestimmung von IgG-Antikörpern wurde das eines Patienten mit Psoriasis rheumatica, zur Bestimmung der IgM-Antikörper das eines Patienten mit Erythema exudativum multiforme verwendet. Die Bestimmung der gebundenen Antikörper erfolgte wie unter Beispiel 1 beschrieben.

Die Ergebnisse, aufgeführt in **Tabelle 2**, belegen, daß unabhängig von der absoluten Größe des Meßsignals durch geeignete Wahl der Lipocortinkonzentration bei der Beschichtung eine maximale Reaktion erzielt werden kann. Unter den in diesem Beispiel gewählten Versuchbedingungen wurde eine Sättigung des Signals, sowohl der Kontrolle als auch der pathologischen Proben, bei einer Lipocortinkonzentration größer 2 mg/l erzielt.

### Beispiel 4

### Beeinflussung des Signal/Hintergrund-Verhältnisses durch Wahl des zur Probenverdünnung eingesetzten Pufferlösung

Mikrotitrationsplatten wurden wie in Beispiel 1 beschrieben mit PP4 und Lipocortin I/II-Gemisch in einem Acetat-Puffer bei pH 5,5 beschichtet und wie in Beispiel 2 beschrieben mit Rinderserumalbumin nachbeschichtet. Humanes Serum aus einem Normalspenderpool und das Serum eines Patienten mit systemischem Lupus erythematodes wurden in einem Tris-Puffer (0,05 mol/l Tris, pH 7,4, enthaltend Rinderserumalbumin 3% (w/w), ®Tween 20 0,5% (w/w)) und verschiedenen Konzentrationen NaCl (0,045, 0,1, 0,2, 0,3, 0,4, 0,5, 0,6 und 0,8 mol/l) im Verhältnis 1:20 verdünnt. Die Bestimmung der gebundenen Antikörper vom IgG-Typ erfolgte wie unter Beispiel 1 beschrieben.

Die in **Tabelle 3** dargestellten Ergebnisse belegen, daß die Zusammensetzung der zur Probenverdünnung eingesetzten Pufferlösung (z.B. Zusatz von NaCl) zu einer erheblichen Reduktion des Hintergrundsignals führen kann, während das Signal in einer pathologischen Probe weniger stark beeinflußt wird. Dadurch läßt sich ein Optimum des Signal/Hintergrund-Verhältnisses erzielen. In diesem Beispiel wurde durch Zusatz von NaCl bei der Bestimmung von Antikörpern vom IgG-Typ gegen PP4 ein Optimum bei einer Konzentration von ca. 0,3 mol/l erreicht; gegen ein Gemisch von Lipocortin I und Lipocortin II lag das Optimum bei ca. 0,2 mol/l. Bei einer Optimierung eines Testsystems zur Erfassung von Antikörpern gegen Lipocortine ist daher nicht nur die Beschichtung der Festphase von Bedeutung, sondern auch die Wahl eines geeigneten Puffers zur Verdünnung und Inkubation der Sera auf der Festphase spielt eine entscheidende Rolle.

### Beispiel 5

### Suppression unspezifischer Absorption der anti-human Antikörper-POD Konjugate an Proteine der Festphase

Mikrotitrationsplatten wurden wie in Beispiel 1 beschrieben mit PP4 und PP4-X in einem Acetat-Puffer bei pH 5,5 beschichtet und wie in Beispiel 2 beschrieben mit Rinderserumalbumin nachbeschichtet. Humanes Serum aus einem Normalspenderpool und die Sera von Patienten mit systemischem Lupus erythematodes (für die anti-PP4 Bestimmung) bzw. einem Melanom (für die anti-PP4-X Bestimmung) wurden in einem Tris-Puffer (0,05 mol/l Tris, pH 7,4, enthaltend Rinderserumalbumin 3% (w/w), Tween 20 0,5% (w/w)) und 0,9% NaCl) im Verhältnis 1:10 verdünnt. In Abweichung zu unter Beispiel 1 beschriebenen Bestimmung der Antikörper gegen PP4 und PP4-X, wurde zur Verdünnung der anti-hIgG-POD und anti-hIgM-POD Konjugatkonzentrate ein Tris-Puffer (0,05 mol/l Tris, pH 7,4, enthaltend Rinderserumalbumin 1% (w/w), ®Tween 20 0,5% (w/w) und NaCl 0,045 mol/l) verwendet, der mit verschiedenen Konzentrationen (0, 0,01, 0,02, 0,03 und 0,04 % w/w) eines monoklonalen Antikörpers (z.B. Bestell Nr. NTIDYO 80/63, Behringwerke Marburg) aufgestockt wurde.

Die Ergebnisse in **Tabelle 4** belegen, daß durch mit steigender Zugabe von IgG aus Maus (z.B. monoklonalen Antikörper, Bestell Nr. NTIDYO 80/63, Behringwerke Marburg) das Signal sowohl für den Pool aus Normalsera, als auch für die pathologischen Sera verringert wird. Das Verhältnis zwischen beiden (Pathologisch/Normal) bleibt bei der anti-PP4 Bestimmung annähernd gleich, bei PP4-X steigt der numerische Wert an, was aber auf Grund des niedrigen Signals in den Normalsera in diesem Fall wohl rein rechnerisch bedingt ist. Dieses Beispiel zeigt somit, daß durch geeignete Wahl von Zusätzen in Konjugatverdünnungsmedien unspezifische Wechselwirkungen zwischen dem Konjugatantikörper und den Proteinen an der Festphase supprimiert werden können.

### Beispiel 6

### Suppression durch Rheumafaktoren verursachter, falsch positiver IgM-Bestimmungen

Mikrotitrationsplatten wurden wie in Beispiel 1 beschrieben mit PP4-X in einem Acetat-Puffer bei pH 5,5 beschichtet und wie in Beispiel 2 beschrieben mit Rinderserumalbumin nachbeschichtet. Humane Sera mit niedrigen Titern an anti-PP4-X Antikörpern vom IgG- und IgM-Typ (IgG-/IgM-) und hohen Titern nur vom IgG-(IgG+/-IgM-) bzw. IgM-Typ (IgG-/IgM+) wurden wie in Beispiel 4 beschrieben in einem Verhältnis von 1:5 in Probenpuffer vorverdünnt. In einem ersten Versuchsansatz wurden die vorverdünnten Sera mit gleichen Volumina eines in unterschiedlichen Konzentrationen in Probenpuffer verdünnten, Rheumafaktor-haltigen Serums (z.B. Rheumafaktor Positivkontrolle BW 18683 K.Nr. 12; 125 IU/ml; Behringwerke, Marburg) gemischt (Endkonzentrationen des Rheumaserums 1:160, 1:80, 1:40, 1:20, 1:10 und 1:5). In jeweils 0,1 ml dieser Mischungen wurde wie in Beispiel 1 beschrieben der Gehalt an Antikörpern gegen PP4-X bestimmt.

Aus den in **Tabelle 5** dargestellten, um die Messwerte im Rheumafaktor-haltigen Serum korrigierten Ergebnissen ist ersichtlich, daß in einem humanen Serum mit hohem Titer an Antikörpern vom IgG-Typ, an diese IgG-Antikörper gebundener "Rheumafaktor" (= anti-humanIgG IgM-Antikörper) bei der IgM-Bestimmung mit erfaßt wird. Sind die Titer an anti-Lipocortin IgG-Antikörpern gering ("IgG-"), so ist die IgM-Bestimmung unbeeinflußt und unabhängig vom Titer an anti-Lipocortin IgM-Antikörpern ("IgM-" oder "IgM+").

In einem zweiten Versuch wurde das "IgG+/IgM-" Serum, das mit Rheumafaktor falsch erhöhte IgM-Titer anzeigte (siehe Tabelle 5) wie oben beschrieben mit dem Rheumafaktorhaltigen Serum gemischt. Die Endkonzentration des "IgG+/IgM-" Serums betrug 1:10, des RF-Serums 1:20. Zur Verdünnung wurde der unter Beispiel 4 beschriebene Probenpuffer verwendet, der mit unterschiedlichen Anteilen (1:160 bis 1:10) einer Lösung der Gamma-Globulinfraktion von Kaninchen anti-Schaferythrozyten Antikörpern (z.B. Ambozeptor für Rapitex Reagenzien, Nr. 62 MH, Charge 08, Behringwerke, Marburg) gemischt worden war. Die Bestimmung der IgM-Titer wurde wie oben beschrieben durchgeführt.

Die ebenfalls in Tabelle 5 aufgezeigten Ergebnisse belegen, daß durch Zusatz einer Lösung einer Gamma-Globulinfraktion in das zur Probenverdünnung benutzte Medium, die durch Bindung von Rheumafaktor an IgG an der Festphase falsch erhöhten IgM-Bestimmungen neutralisiert werden kann.

### Beispiel 7

### Screening von Sera unter Verwendung einer mit allen Lipocortinen beschichteten Festphase

Ein Panel an humanen Sera von normalen Spendern und von Patienten mit autoimmunologischen und/oder entzündlichen Erkrankungen wurde wie in Beispiel 6 beschrieben in Verdünnungen von 1:10 auf Antikörper vom IgG und IgM-Typ gegen Lipocortine getestet. Die Mikrotitrationsplatten wurden wie in Beispiel 1 beschrieben mit PP4, PP4-X, PAP III, p68 und Lipocortin I/II-Gemisch, sowie mit einem Gemisch aus allen Lipocortinen mit einer Konzentration von 5 mg/l (Lipocortin II: 1,25 mg/l) beschichtet und wie in Beispiel 2 beschrieben mit Rinderserumalbumin nachbeschichtet.

Die Ergebnisse der Bestimmungen der IgG- und IgM-anti-Lipocortinantikörper in Seren von 114 normalen Blutspendern sind in **Tabelle 6** aufgeführt. Die Auflistung zeigt die Häufigkeit der gefundenen Meßwerte in logarithmisch eingeteilten Klassen (in mExtinktionen). Die Mittelwerte und Mediane der jeweiligen Verteilungen stimmen gut überein, sind jedoch für jedes Beschichtungsantigen verschieden.

In 123 Proben von Patienten mit autoimmunologischen und/oder entzündlichen Erkrankungen wurden 69 gefunden, deren Antikörpertiter gegen Lipocortine vom IgG- bzw. IgM-Typ oberhalb des 90%-Intervalls der oben jeweils bestimmten "Normalverteilung" lagen und im folgenden als "positiv" bezeichnet werden. Es zeigte sich, daß sich bei 38 Patienten Antikörper vom IgG-Typ und bei 25 Patienten von IgM-Typ gebildet hatten, die nur mit einem Lipocortin reagierten. Die Spezifität verteilte sich dabei auf alle Lipocortine (siehe **Tabelle 7**). Dies zeigt, daß nur ein umfassendes Screening mit allen Lipocortinen und gegen beide Antikörpertypen in der Lage ist durch anti-Lipocortin Antikörper bedingte Störungen in der Funktion der Lipocortine zu erfassen.

43 positive Proben wurden auch auf einer mit allen Lipocortinen beschichteten Mikrotitrationsplatte getestet. Dabei wurden 13/20 mit IgG- bzw. 7/15 mit IgM-Antikörpern als "positiv" erkannt (siehe **Tabelle 7**). Dieses Beispiel belegt somit, daß eine mit allen Lipocortinen beschichtete Festphase zu einem generellen Screeningverfahren von Proben auf Antikörper sowohl vom IgG- als auch vom IgM-Typ gegen einzelne als auch gegen mehrere Lipocortine geeignet ist.

**Tabelle 1:**

| | | | | |
|---|---|---|---|---|
| Auswirkung der Nachbeschichtung von mit Lipocortin I/II-Gemisch beschichteten Mikrotitrationsplatten auf die IgG- und IgM-Bestimmung in einem humanen Serumpool von Normalspendern (Beispiel 2). | | | | |
| Zur Nachbeschichtung wurden 1% Lösungen von Rinderserumalbumin (RSA), Ovalbumin (OA), Gelatin (G), Fötales Kälberserum (FCS), sowie 0,1% Kaninchen IgG (KI) und Beschichtungspuffer ohne Zusatz (-) von Protein verwendet mit pH-Werten von 5,5, 7,5 und 9,5. Das Serum wurde 1:10 in Puffer verdünnt eingesetzt. Angegeben sind die Meßwerte in mExtinktionen; Ak = Antikörperklasse. | | | | |

| Protein pH-Wert bei Nachbeschichtung | | | | Ak |
|---|---|---|---|---|
| | 5,5 | 7,5 | 9,5 | |
| | | | | IgG |
| - | 18 | 29 | 24 | |
| RSA | 10 | 17 | 20 | |
| OA | 250 | 126 | 92 | |
| G | 21 | 22 | 19 | |
| FCS | 38 | 55 | 45 | |
| KI | 24 | 12 | 36 | |
| | | | | IgM |
| - | 70 | 86 | 93 | |
| RSA | 64 | 74 | 78 | |
| OA | 73 | 85 | 87 | |
| G | 111 | 107 | 99 | |
| FCS | 56 | 84 | 90 | |
| KI | 301 | 160 | 131 | |

**Tabelle 2:**

| | | | | | |
|---|---|---|---|---|---|
| Abhängigkeit der anti-PP4 und -PP4-X IgG- und IgM--Bestimmung in Sera von normalen Blutspendern (N) und von Patienten mit Autoimmunerkrankungen (P) von der Konzentration des Beschichtungsantigens. Angegeben sind die Meßwerte in mExtinktionen (Beispiel 3). | | | | | |

| Konzentration [mg/l] | Beschichtungsantigen | | | | Ak-Klasse |
|---|---|---|---|---|---|
| | PP4 | | PP4-X | | |
| | N | P | N | P | |
| 0,04 | 63 | 92 | 69 | 104 | IgG |
| 0,08 | 53 | 101 | 70 | 101 | |
| 0,16 | 50 | 93 | 71 | 101 | |
| 0,31 | 62 | 96 | 86 | 109 | |
| 0,63 | 78 | 120 | 110 | 136 | |
| 1,25 | 122 | 166 | 139 | 165 | |
| 2,5 | 142 | 187 | 180 | 207 | |
| 5,0 | 152 | 201 | 185 | 208 | |
| 0,04 | 10 | 119 | 23 | 64 | IgM |
| 0,08 | 25 | 64 | 22 | 64 | |
| 0,16 | 22 | 65 | 26 | 67 | |
| 0,31 | 25 | 72 | 29 | 77 | |
| 0,63 | 28 | 72 | 35 | 94 | |
| 1,25 | 37 | 97 | 46 | 149 | |
| 2,5 | 42 | 118 | 62 | 178 | |
| 5,0 | 46 | 129 | 63 | 207 | |

**Tabelle 3:**

| | | | | | | |
|---|---|---|---|---|---|---|
| Einfluß von NaCl im Probenverdünnungspuffer auf die Bestimmung von Antikörpern vom IgG-Typ gegen PP4 und Lipocortin I/II-Gemisch (L I/II) in einem Pool von Seren von Normalspendern (Normalsera) und Patienten mit systemischem Lupus erythematodes (Pathoserum). Angegeben sind die Meßwerte in mExtinktionen (Beispiel 4). | | | | | | |

| NaCl-Konzentration [mol/l] | Extinktion (490 nm - 630 nm) | | | | Verhältnis Normalsera/Pathosera | |
|---|---|---|---|---|---|---|
| | Normalsera | | Pathoserum | | | |
| | PP4 L I/II | | PP4 L I/II | | PP4 L I/II | |
| 0,045 | 194 | 152 | 627 | 848 | 3,2 | 2,5 |
| 0,1 | 106 | 11 | 549 | 791 | 5,2 | 3,1 |
| 0,2 | 62 | 85 | 406 | 587 | 6,5 | 3,3 |
| 0,3 | 40 | 76 | 270 | 439 | 6,8 | 3,0 |
| 0,4 | 37 | 64 | 180 | 352 | 4,9 | 2,6 |
| 0,5 | 36 | 62 | 132 | 282 | 3,7 | 2,1 |
| 0,6 | 38 | 61 | 95 | 218 | 2,5 | 1,6 |
| 0,8 | 33 | 49 | 79 | 134 | 2,4 | 1,5 |

**Tabelle 4:**

| | | | | | | |
|---|---|---|---|---|---|---|
| Einfluß eines monoklonalen Antikörpers im Konjugatverdünnungspuffer auf die Bestimmung von IgG-Antikörpern gegen PP4 und PP4-X in einem Pool humaner Sera von Normalspendern (Normalsera) und von Patienten mit systemischem Lupus erythematodes (PP4) bzw. einem Melanom (PP4-X) (Pathosera). Angegeben sind die Meßwerte in mExtinktionen (Beispiel 5). | | | | | | |

| MAk-Konzentration [%] | Extinktion (490 nm - 630 nm) | | | | Verhältnis Normalsera/Pathosera | |
|---|---|---|---|---|---|---|
| | Normalsera | | Pathoserum | | | |
| | PP4 | PP4-X | PP4 | PP4-X | PP4 | PP4-X |
| 0 | 169 | 64 | 585 | 336 | 3,5 | 5,3 |
| 0,01 | 75 | 20 | 246 | 209 | 3,3 | 10,3 |
| 0,02 | 58 | 13 | 181 | 168 | 3,1 | 13,1 |
| 0,03 | 49 | 9 | 184 | 152 | 3,7 | 16,3 |
| 0,04 | 40 | 6 | 132 | 134 | 3,3 | 22,9 |

**Tabelle 5:**

| | | | | | | |
|---|---|---|---|---|---|---|
| Suppression durch Rheumafaktor verursachter, falsch positiver IgM-Bestimmungen. Sera mit niedrigen Titern an anti-PP4-X Antikörpern vom IgG- und IgM-Typ (IgG-/IgM-) und hohen Titern nur vom IgG (IgG+/IgM-) bzw. IgM-Typ (IgG-/IgM+), sowie Probenpuffer wurden mit unterschiedlichen Mengen eines Rheumafaktor-haltigen Humanserums (125 IU/ml) gemischt. Aufgeführt sind die IgG- und IgM- Bestimmung ohne Zusätze, die Messwerte der IgM-Bestimmung nach Zugabe von Rheumafaktorserum sowie bei Zugabe unterschiedlicher Mengen einer Gamma-Globulinlösung aus Kaninchen zu einem mit Rheumafaktor falsch positiv reagierenden Serum. Angegeben sind die Meßwerte in mExtinktionen (Beispiel 6). | | | | | | |

| | IgG-/IgM- | | | IgG+/IgM- | IgG-/IgM+ | |
|---|---|---|---|---|---|---|
| Messwerte ohne Zusatz [mE] | | | | | | |
| IgG | | 43 | | 721 | | 37 IgM |
| 37 | 91 | | 228 | | | |

| IgM-Bestimmung unter Zusatz von RF-Serum | | | | | | |
|---|---|---|---|---|---|---|
| 1:160 | | 26 | | 93 | 200 | |
| 1:80 | | 42 | | 105 | 208 | |
| 1:40 | | 32 | | 111 | 231 | |
| 1:20 | | 34 | | 149 | 250 | |
| 1:10 | | 24 | | 176 | 215 | |
| 1:5 | | 18 | | 201 | 209 | |

| IgM-Bestimmung unter Zusatz von Gamma-Globulinlösung | | | | IgG +/IgM+ RF-Serum (1:20) | | |
|---|---|---|---|---|---|---|
| ohne | | | | 157 | | |
| 1:160 | | | | 147 | | |
| 1:80 | | | | 126 | | |
| 1:40 | | | | 140 | | |
| 1:20 | | | | 111 | | |
| 1:10 | | | | 112 | | |

**Tabelle 6:**

| | | | | | | |
|---|---|---|---|---|---|---|
| Statistische Analyse der Verteilung der Antikörpertiter vom IgG- bzw. IgM-Typ gegen einzelne und gegen Gemische von Lipocortinen in einem Panel von Sera von Normalspendern (n=114) in logarithmisch unterteilten Klassen (mExtinktionen) (Beispiel 7). | | | | | | |

| | PP4 | PP4-X | PAPIII | p68 | LI/II | PP4-LII |
|---|---|---|---|---|---|---|
| IgG-Typ: | | | | | | |
| Anzahl | 114 | 114 | 114 | 114 | 114 | 114 |
| Mittelwert | 42 | 76 | 47 | 80 | 46 | 58 |
| Median | 39 | 67 | 41 | 78 | 49 | 53 |
| 90% Intervall | 90 | 117 | 105 | 193 | 109 | 112 |

| IgM-Typ: | | | | | | |
|---|---|---|---|---|---|---|
| Anzahl | 114 | 114 | 114 | 114 | 114 | 114 |
| Mittelwert | 41 | 70 | 43 | 59 | 62 | 58 |
| Median | 40 | 69 | 43 | 61 | 63 | 61 |
| 90% Intervall | 93 | 154 | 98 | 141 | 136 | 126 |

**Tabelle 7**

| | | | | | | |
|---|---|---|---|---|---|---|
| Vorkommen von Antikörpern vom IgG- und IgM-Typ gegen Lipocortine in Seren von Patienten mit autoimmunologischen und/oder entzündlichen Erkrankungen. Als positiv wurden diejenigen Proben eingestuft, deren Antikörpertiter oberhalb des 90% Intervalls der jeweiligen Verteilung der Meßwerte von Normalspendern (siehe Tabelle 6 und 7) liegen. Mit PP4-L II sind die Ergebnisse der Untersuchung eines Teils dieser Proben auf einer mit allen Lipocortinen beschichteten Mikrotiterplatte bezeichnet; L I/II = Lipocortin I und Lipocortin II-Gemisch (Beispiel 7). Gesamtzahl der Patienten: 123 davon positiv (IgG- und/oder IgM-Typ): 69 davon wurden mit jeweils als positiv gefunden: | | | | | | |

| Antigen | | | | | | |
|---|---|---|---|---|---|---|
| Anti-Körpertyp | PP4 | PP4-X | PAP III | p68 | L I/II | PP4-LII |
| | (Σ=69) | | | | | (Σ=43) |
| IgG | 16%(11) | 15%(10) | 10%(7) | 2%(1) | 13% (9) | 30%(13) |
| IgM | 4% (3) | 4% (3) | 7% (5) | 2%(1) | 19%(13) | 16% (7) |

## Patentansprüche (Patentansprüche für folgende(n) Vertragsstaat(en): AT, BE, CH, SE, DE, FR, GB, IT, LI, LU, NL)

1. Heterogener Immunoassay zur Bestimmung von Antikörpern gegen Lipocortine, dadurch gekennzeichnet, daß als an die Festphase gebundener bioaffiner Bindungspartner ein Gemisch aus den bekannten Lipocortinen PP4, PP4-X, PAPIII, Lipocortin I und II verwendet wird.

2. Immunoassay gemäß Anspruch 1, dadurch gekennzeichnet, daß als markierter bioaffiner Bindungspartner ein Antikörper oder Antikörpergemisch gegen mindestens eine humane Antikörperklasse eingesetzt ist.

3. Immunoassay nach Anspruch 1, dadurch gekennzeichnet, daß der Bindungspartner an der Festphase Proteine aus der Lipocortinfamilie der gleichen Spezies sind von der die Körperflüssigkeit stammt, in der die Antikörper gegen diese Proteine bestimmt werden sollen.

4. Immunoassay nach Anspruch 1, dadurch gekennzeichnet, daß die Beschichtung der Festphase mit einem oder mehreren Proteinen aus der Lipocortinfamilie im pH-Bereich von 5 bis 10, bevorzugt im pH-Bereich von 5 bis 7, besonders bevorzugt bei pH 5,5 erfolgt.

5. Immunoassay nach Anspruch 4, dadurch gekennzeichnet, daß die Beschichtung der Festphase mit Proteinen aus der Lipocortinfamilie unter Zusatz von divalenten Kationen erfolgt, besonders bevorzugt unter Zusatz von CaCl₂.

6. Immunoassay nach Anspruch 1, dadurch gekennzeichnet, daß die Körperflüssigkeit in einem Puffermedium verdünnt wird, das Reagenzien enthält, das die Bindung von in der Körperflüssigkeit enthaltenen Rheumafaktoren an Antikörper dieser Körperflüssigkeit verhindert, bevorzugt werden dabei Gamma-Globulin-Fraktionen aus Kaninchenserum, besonders bevorzugt solche aus Kaninchen, die zuvor gegen Schafterythrozyten immunisiert wurden.

7. Immunoassay nach Anspruch 1, dadurch gekennzeichnet, daß der markierte Bindungspartner ein gegen IgG, IgM oder die schwere Kette von IgG und IgM gerichteter Antikörper ist.

8. Immunoassay nach Anspruch 1, dadurch gekennzeichnet, daß die Markierung ein Enzym ist, bevorzugterweise eine Peroxidase.

9. Verfahren zur Beschichtung von Festphasen für heterogene Immunoassays zur Bestimmung von Antikörpern gegen Lipocortine, dadurch gekennzeichnet, daß die Beschichtung mit einem Gemisch aus den bekannten Lipocortinen PP4, PP4-X, PAPIII und Lipocortin I und II in einem pH Bereich von 5 - 10, gegebenenfalls in Gegenwart von zweiwertigen Kationen, wie z. B. Ca²⁺, erfolgt.

## Patentansprüche (Patentansprüche für folgende(n) Vertragsstaat(en): ES)

1. Heterogener Immunoassay zur Bestimmung von Antikörpern gegen Lipocortine, dadurch gekennzeichnet, daß als an die Festphase gebundener bioaffiner Bindungspartner ein Gemisch aus den bekannten Lipocortinen PP4, PP4-X, PAPIII, Lipocortin I und II verwendet wird.

2. Immunoassay gemäß Anspruch 1, dadurch gekennzeichnet, daß als markierter bioaffiner Bindungspartner ein Antikörper oder Antikörpergemisch gegen mindestens eine humane Antikörperklasse eingesetzt ist.

3. Immunoassay nach Anspruch 1, dadurch gekennzeichnet, daß der Bindungspartner an der Festphase Proteine aus der Lipocortinfamilie der gleichen Spezies sind von der die Körperflüssigkeit stammt, in der die Antikörper gegen diese Proteine bestimmt werden sollen.

4. Immunoassay nach Anspruch 1, dadurch gekennzeichnet, daß die Beschichtung der Festphase im pH-Bereich von 5 bis 10, bevorzugt im pH-Bereich von 5 bis 7, besonders bevorzugt bei pH 5,5 erfolgt.

5. Immunoassay nach Anspruch 4, dadurch gekennzeichnet, daß die Beschichtung der Festphase unter Zusatz von divalenten Kationen erfolgt, besonders bevorzugt unter Zusatz von CaCl₂.

6. Immunoassay nach Anspruch 1, dadurch gekennzeichnet, daß die Körperflüssigkeit in einem Puffermedium verdünnt wird, das Reagenzien enthält, die die Bindung von in der Körperflüssigkeit enthaltenen Rheumafaktoren an Antikörper dieser Körperflüssigkeit verhindern, bevorzugt werden dabei Gamma-Globulin-Fraktionen aus Kaninchenserum, besonders bevorzugt solche aus Kaninchen, die zuvor gegen Schaferythrozyten immunisiert wurden.

7. Verfahren nach Anspruch 1, dadurch gekennzeichnet, daß der markierte Bindungspartner ein gegen einzelne oder Gruppen von Immunglobulinklassen gerichteter Antikörper ist.

8. Verfahren nach Anspruch 1, dadurch gekennzeichnet, daß der markierte Bindungspartner ein gegen IgG, IgM oder die schwere Kette von IgG und IgM gerichteter Antikörper ist.

9. Immunoassay nach Anspruch 1, dadurch gekennzeichnet, daß die Markierung ein Enzym ist, bevorzugterweise eine Peroxidase.

10. Verfahren zur Beschichtung von Festphasen für heterogene Immunoassays zur Bestimmung von Antikörpern gegen Lipocortine, dadurch gekennzeichnet, daß die Beschichtung mit einem Gemisch aus den bekannten Lipocortinen PP4, PP4-X, PAPIII und Lipocortin I und II in einem pH Bereich von 5 - 10, gegebenenfalls in Gegenwart von zweiwertigen Kationen, wie z. B. Ca²⁺, erfolgt.

## Claims (Claims for the following Contracting State(s): AT, BE, CH, DE, FR, GB, IT, LI, LU, NL, SE,)

1. A heterogeneous immunoassay for the determination of antibodies against lipocortins, which comprises using a mixture of the known lipocortins PP4, PP4-X, PAPIII, lipocortin I and II as bioaffinity binding partner bound to the solid phase.

2. An immunoassay as claimed in claim 1, wherein an antibody or mixture of antibodies against at least one human antibody class is employed as labeled bioaffinity binding partner.

3. An immunoassay as claimed in claim 1, wherein the binding partner on the solid phase are proteins from the lipocortin family from the same species from which is derived the body fluid in which the antibodies against these proteins are to be determined.

4. An immunoassay as claimed in claim 1, wherein the solid phase is coated with one or more proteins from the lipocortin family in the pH range from 5 to 10, preferably in the pH range from 5 to 7, particularly preferably at pH 5.5.

5. An immunoassay as claimed in claim 4, wherein the solid phase is coated with proteins from the lipocortin family with the addition of divalent cations, particularly preferably with the addition of CaCl₂.

6. An immunoassay as claimed in claim 1, wherein the body fluid is diluted in a buffer medium which contains reagents which prevent the binding of rheumatoid factors contained in the body fluid to antibodies in this body fluid, preferred in this context being gamma-globulin fractions from rabbit serum, particularly preferably those from rabbits which have previously been immunized against sheep erythrocytes.

7. An immunoassay as claimed in claim 1, wherein the labeled binding partner is an antibody directed against IgG, IgM or the heavy chain of IgG and IgM.

8. An immunoassay as claimed in claim 1, wherein the labeling is an enzyme, preferably a peroxidase.

9. A method for coating solid phases for heterogeneous immunoassays for the determination of antibodies against lipocortins, which comprises carrying out the coating with a mixture of the known lipocortins PP4, PP4-X, PAPIII and lipocortin I and II in a pH range of 5-10, where appropriate in the presence of divalent cations such as, for example, Ca²⁺.

## Claims (Claims for the following Contracting State(s): ES)

1. A heterogeneous immunoassay for the determination of antibodies against lipocortins, which comprises using a mixture of the known lipocortins PP4, PP4-X, PAPIII, lipocortin I and II as bioaffinity binding partner bound to the solid phase.

2. An immunoassay as claimed in claim 1, wherein an antibody or mixture of antibodies against at least one human antibody class is employed as labeled bioaffinity binding partner.

3. An immunoassay as claimed in claim 1, wherein the binding partner on the solid phase are proteins from the lipocortin family from the same species from which is derived the body fluid in which the antibodies against these proteins are to be determined.

4. An immunoassay as claimed in claim 1, wherein the solid phase is coated in the pH range from 5 to 10, preferably in the pH range from 5 to 7, particularly preferably at pH 5.5.

5. An immunoassay as claimed in claim 4, wherein the solid phase is coated with the addition of divalent cations, particularly preferably with the addition of CaCl₂.

6. An immunoassay as claimed in claim 1, wherein the body fluid is diluted in a buffer medium which contains reagents which prevent the binding of rheumatoid factors contained in the body fluid to antibodies in this body fluid, preferred in this context being gamma-globulin fractions from rabbit serum, particularly preferably those from rabbits which have previously been immunized against sheep erythrocytes.

7. A method as claimed in claim 1, wherein the labeled binding partner is an antibody directed against single or groups of immunoglobulin classes.

8. A method as claimed in claim 1, wherein the labeled binding partner is an antibody directed against IgG, IgM or the heavy chain of IgG and IgM.

9. An immunoassay as claimed in claim 1, wherein the labeling is an enzyme, preferably a peroxidase.

10. A method for coating solid phases for heterogeneous immunoassays for the determination of antibodies against lipocortins, which comprises carrying out the coating with a mixture of the known lipocortins PP4, PP4-X, PAPIII and lipocortin I and II in a pH range of 5-10, where appropriate in the presence of divalent cations such as, for example, Ca²⁺.

## Revendications (Revendications pour l'(les) Etat(s) contractant(s) suivant(s): AT, BE, CH, DE, FR, GB, IT, LI, LU, NL, SE)

1. Essai immunologique hétérogène pour la détermination d'anticorps dirigés contre des lipocortines, caractérisé en ce que l'on utilise comme partenaire de liaison doté de bioaffinité, fixé sur la phase solide, un mélange des lipocortines connues PP4, PP4-X, PAP III, lipocortine I et lipocortine II.

2. Essai immunologique selon la revendication 1, caractérisé en ce que l'on utilise comme partenaire de liaison doté de bioaffinité, marqué, un anticorps ou mélange d'anticorps dirigé contre au moins une classe d'anticorps humains.

3. Essai immunologique selon la revendication 1, caractérisé en ce que le partenaire de liaison à la phase solide consiste en des protéines de la famille des lipocortines de la même espèce dont provient le liquide corporel dans lequel doivent être déterminés les anticorps dirigés contre ces protéines.

4. Essai immunologique selon la revendication 1, caractérisé en ce que l'on effectue le revêtement de la phase solide avec une ou plusieurs protéines de la famille des lipocortines dans l'intervalle de pH allant de 5 à 10, de préférence dans l'intervalle de pH allant de 5 à 7, en particulier à pH 5,5.

5. Essai immunologique selon la revendication 4, caractérisé en ce que le revêtement de la phase solide avec les protéines de la famille des lipocortines s'effectue avec addition de cations divalents, de façon particulièrement préférée avec addition de CaCl₂.

6. Essai immunologique selon la revendication 1, caractérisé en ce que le liquide corporel est dilué dans un milieu tampon qui contient des réactifs et qui empêche la liaison de facteurs rhumatismaux, contenus dans le liquide corporel, à des anticorps de ce liquide corporel, et de préférence on utilise à cet effet des fractions de γ-globulines provenant de sérum de lapin, de façon particulièrement préférée de sérum de lapins qui ont été préalablement immunisés contre des érythrocytes de mouton.

7. Essai immunologique selon la revendication 1, caractérisé en ce que le partenaire de liaison marqué est un anticorps dirigé contre une IgG, une IgM ou la chaîne lourde d'IgG et d'IgM.

8. Essai immunologique selon la revendication 1, caractérisé en ce que le marqueur est une enzyme, de préférence une peroxydase.

9. Procédé pour le revêtement de phase solide pour des essais immunologiques hétérogènes destinés à la détermination d'anticorps dirigés contre des lipocortines, caractérisé en ce que le revêtement est effectué avec un mélange des lipocortines connues PP4, PP4-X, PAP III, lipocortine I et lipocortine II, dans un intervalle de pH allant de 5 à 10, éventuellement en présence de cations divalents, comme par exemple Ca²⁺.

## Revendications (Revendications pour l'(les) Etat(s) contractant(s) suivant(s): ES)

1. Essai immunologique hétérogène pour la détermination d'anticorps dirigés contre des lipocortines, caractérisé en ce que l'on utilise comme partenaire de liaison doté de bioaffinité, fixé sur la phase solide, un mélange des lipocortines connues PP4, PP4-X, PAP III, lipocortine I et lipocortine II.

2. Essai immunologique selon la revendication 1, caractérisé en ce que l'on utilise comme partenaire de liaison doté de bioaffinité, marqué, un anticorps ou mélange d'anticorps dirigé contre au moins une classe d'anticorps humains.

3. Essai immunologique selon la revendication 1, caractérisé en ce que le partenaire de liaison à la phase solide consiste en des protéines de la famille des lipocortines de la même espèce dont provient le liquide corporel dans lequel doivent être déterminés les anticorps dirigés contre ces protéines.

4. Essai immunologique selon la revendication 1, caractérisé en ce que le revêtement de la phase solide s'effectue dans l'intervalle de pH allant de 5 à 10, de préférence dans l'intervalle de pH allant de 5 à 7, de façon particulièrement préférée à pH 5,5.

5. Essai immunologique selon la revendication 4, caractérisé en ce que le revêtement de la phase solide s'effectue avec addition de cations divalents, en particulier avec addition de CaCl₂.

6. Essai immunologique selon la revendication 1, caractérisé en ce que le liquide corporel est dilué dans un milieu tampon qui contient des réactifs et qui empêche la liaison de facteurs rhumatismaux, contenus dans le liquide corporel, à des anticorps de ce liquide corporel, et de préférence on utilise à cet effet des fractions de γ-globulines provenant de sérum de lapin, de façon particulièrement préférée de sérum de lapins qui ont été préalablement immunisés contre ces érythrocytes de mouton.

7. Procédé selon la revendication 1, caractérisé en ce que le partenaire de liaison marqué est un anticorps dirigé contre des classes d'immunoglobulines individuelles ou des groupes de classes d'immunoglobulines.

8. Procédé selon la revendication 1, caractérisé en ce que le partenaire de liaison marqué est un anticorps dirigé contre une IgG, une IgM ou la chaîne lourde d'IgG et d'IgM.

9. Essai immunologique selon la revendication 1, caractérisé en ce que le marqueur est une enzyme, de préférence une peroxydase.

10. Procédé pour le revêtement de phase solide pour des essais immunologiques hétérogènes destinés à la détermination d'anticorps dirigés contre des lipocortines, caractérisé en ce que le revêtement est effectué avec un mélange des lipocortines connues PP4, PP4-X, PAP III, lipocortine I et lipocortine II, dans un intervalle de pH allant de 5 à 10, éventuellement en présence de cations divalents, comme par exemple Ca²⁺.
